(19) Europäisches Patentamt
European Patent Office
Office européen des brevets

(11) Veröffentlichungsnummer: **0 336 148**
**A2**

(12) **EUROPÄISCHE PATENTANMELDUNG**

(21) Anmeldenummer: **89104505.6**

(51) Int. Cl.4: **A61L 27/00**

(22) Anmeldetag: **14.03.89**

(30) Priorität: **02.04.88 DE 3811231**
**02.04.88 DE 8804423 U**

(43) Veröffentlichungstag der Anmeldung:
**11.10.89 Patentblatt 89/41**

(84) Benannte Vertragsstaaten:
**AT BE CH DE ES FR GB IT LI LU NL SE**

(71) Anmelder: **Beiersdorf Aktiengesellschaft**
**Unnastrasse 48**
**D-2000 Hamburg 20(DE)**

(72) Erfinder: **Heimerl, Albert, Dr.**
**Immenhorstweg 70**
**D-2000 Hamburg 66(DE)**
Erfinder: **Pietsch, Hanns, Dr.**
**Mittelweg 25**
**D-2000 Hamburg 13(DE)**
Erfinder: **Rademacher, Karl-Heinz, Dr.**
**Ernststrasse 22**
**D-2000 Hannover 71(DE)**
Erfinder: **Schwengler, Helge**
**Kastanienallee 3a**
**D-2084 Rellingen 1(DE)**
Erfinder: **Winkeltau, Gerd, Dr.**
**Wilhelmstrasse 24**
**D-5100 Aachen(DE)**
Erfinder: **Treutner, Karl-Heinz, Dr.**
**Hans-Böckler-Allee 155**
**D-5100 Aachen(DE)**

(54) **Chirurgische Implantate.**

(57) Resorbierbares chirurgisches Implantat zur Unterstützung der Organ- und Gewebsheilung, dadurch gekennzeichnet, daß es

a) ein Homo- oder Copolymerisat aus 3-Hydroxybutansäure (HB) und 3-Hydroxypentansäure (HP, gegebenenfalls im Gemisch mit anderen resorbierbaren polymeren Substanzen,

b) gegebenenfalls chirurgische und/oder medizinische Hilfs- und Zusatzstoffe

enthält.

Fig.1

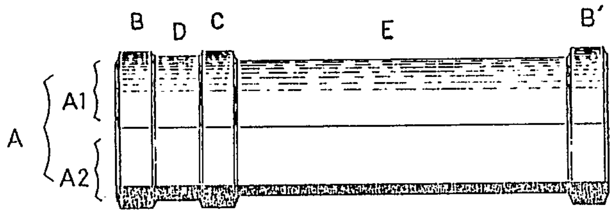


EP 0 336 148 A2

## Chirurgische Implantate

Die Erfindung betrifft resorbierbare chirurgische Implantate, insbesondere zur Unterstützung der Gewebs-und Organheilung.

Derartige Hilfsmittel finden vorzugsweise Verwendung als vom menschlichen und tierischen Organismus abbaubare Schienungshülsen für Gefäße und Hohlorgane sowie als Trennfolien für unterschiedliche Gewebetypen.

Eine weitere Anwendungsmöglichkeit besteht darin, derartige Hilfsmittel mit physiologisch wirksamen Substanzen zu dotieren, um sie damit zu einem inkorporierten, kontrolliert wirksamen Wirkstoffdepot zu machen.

Resorbierbare Materialien und deren Gebrauch zur Herstellung chirurgischer und medizinischer Hilfsmittel sind an sich bekannt. Ihr Vorteil beruht darauf, daß diese nicht postoperativ in einem zweiten riskanten Eingriff aus dem Körper des Patienten entfernt werden müssen, sondern durch dessen Stoffwechsel allmählich abgebaut werden.

Die im chirurgischen Bereich nahezu ausschließlich verwendeten Materialen sind Homo- oder Copolymerisate aus Milchsäure bzw. Glycolsäure.

Diese Materialien besitzen eine Reihe von Nachteilen.

1) Sie sind stark hydrolyseempfindlich. Daher müssen für ihre Lagerung spezielle Vorkehrungen, also weitgehender Ausschluß von Feuchtigkeit, getroffen werden.

2) Wegen ihrer Hydrolyseempfindlichkeit ist die Resorptionsgeschwindigkeit, besonders bei kleinen Wandstärken, zu groß,

3) Die Verarbeitung durch thermoplastische Kunststoffverarbeitungsverfahren ist sehr aufwendig.

Erkrankungen oder Beschädigungen röhrenförmiger Organe - z.B. Blutgefäße, Luft- und Speiseröhre, Darmtrakt - werden operativ durch Anastomose repariert, wenn die Situation Zertrennung dieser Organe erfordert. So wird beispielsweise bei manchen Operationen von Darmkrebs ein ringförmiges, irreparabel geschädigtes Stück Dickdarm entfernt. Die Verbindung der gesunden Teile des zertrennten Organs wird Anastomose genannt.

Durch den operativen Eingriff wird das betreffende Organ wiederum stark beeinträchtigt. Es besteht ein hohes Risiko, daß eine nicht unterstützte Anastomose von Nahtinsuffizienzen begleitet wird. Problematische Anastomosen im Intestinaltrakt weisen daher Insuffizienzquoten bis zu 40 % auf. Weitere Beispiele sind Notfalleingriffe am Gastrointestinaltrakt und die Operation der chronisch-entzündlichen Darmerkrankung im akuten Schub. Sie weisen etwa 20%ige Insuffizienzraten auf. Höchste Quoten besitzt die chirurgische Intervention beim intestinalen Strahlenspätschaden (bis 40 %). Aber auch bei speziellen Lokalisationen, wie im tiefen Rektum und bei Ösophago-gastralen Anastomosen im Halsbereich finden sich Insuffizienzraten über 20 %.

Die Verwendung hohlzylindrischer Körper zur inneren Schienung von Anastomosen ist an sich bekannt (Dis. Colon Rectum, 28 (1985), 904-7; Br. J. Surg. 71 (1984), 726-729). Sie bewirken eine Unterstützung des geschädigten Organs und gewährleisten, daß der Materialfluß aufrechterhalten und nicht durch Kollabieren des Organs an der Nahtstelle unterbrochen wird. Weiterhin wird die Nahtstelle abgedichtet und Materialfluß in die Umgebung des Organs vermieden.

Bei Verwendung nichtresorbierbarer Anastomoseröhren wird erwartet, daß sich die Hülsen nach Heilung der Anastomose entweder durch einen Zweiteingriff entfernen lassen oder, wie es bei Darmanastomosen möglich ist, nach Resorption des Nahtmaterials durch die Eigenperistaltik des geschienten Organs ausgeschieden werden.

Im letzteren Falle ist die Prognose im allgemeinen nicht optimistisch, da bei der Passage der Röhre durch den Verdauungstrakt Komplikationen auftreten können. So besteht die Gefahr der Verkantung, wodurch ein Ileus verursacht werden kann. Löst sich die Röhre nicht von der Darmwand, muß sie endoskopisch oder operativ entfernt werden.

Wissenschaftlich fundierte Überprüfungen des Prinzips der inneren Schienung liegen erst seit einigen Jahren aus Amerika und Australien vor. Keine Erfahrungen existieren weltweit mit dem Einsatz resorbierbarer Biomaterialien zur inneren Schienung.

Folien aus resorbierbaren Materialien haben in der Chirurgie einen hohen Stellenwert als Schutz von Organen, Nervenbahnen und Sehnen gegen sich neu bildendes Narbengewebe. Dabei dienen die Folien als mechanische Barriere zwischen unabhängigen Gewebstypen bzw. Gewebsschichten. Nichtresorbierbares Material ist für diese Verwendung in hohem Maße ungeeignet, da Entfernen der chirurgischen Folie die separierten Gewebsteile so stark beeinträchtigen würde, daß erneut die Gefahr gegenseitiger Beeinträchtigung oder sogar des Zusammenwachsens bestünde.

Für viele Fälle ist eine einfache resorbierbare Folie nicht ausreichend. So wurde zum Beispiel gefunden, daß eine Einscheidung von Sehnen und Muskulaturgewebe an Knochen Nekrosen der Knochenhaut hervorrufen. Dies beruht auf der Störung

des osmotischen Austauschs zwischen den involvierten Gewebsregionen.

Aufgabe der vorliegenden Erfindung war somit, ein Material zu finden, welches so unempfindlich gegen Hydrolyse ist, daß es ohne Aufwand gelagert werden kann, aber dennoch im menschlichen oder tierischen Organismus in angemessener Zeit abbaubar ist. Dabei muß das Material formstabil sein, darf aber gleichzeitig nicht so spröde -sein, daß es unter peristaltischer Einwirkung zerbirst oder Organreizung hervorruft. Insbesondere sollte es den aggressiven Sekreten des Intestinaltraktes einesteils hinreichend lange widerstehen, letztlich aber doch abgebaut werden. Diese Forderung war bislang von keinem Material erfüllt worden.

Weiterhin sollten chirurgische Hilfsmittel ausgestaltet werden, die im Organismus des Warmblüters die Gewebs- und Organheilung unterstützen, speziell als Implantate wie Anastomoseröhren und Trennfolien. Schließlich sollten Verfahren entwickelt werden, welche auf ökonomische Weise die Herstellung solcher Hilfsmittel ermöglichen.

Es hat sich gezeigt - und darin besteht die Lösung der ersten Aufgabe der Erfindung - daß Homo- und Copolymerisate aus 3-Hydroxybutansäure (HB) und 3-Hydroxypentansäure (HP) - gegebenenfalls im Gemisch mit anderen resorbierbaren Polymeren - über die geforderten Materialeigenschaften verfügen und sich in vorteilhafter Weise zu chirurgischen Implantaten verarbeiten lassen. In EP-OS 204 442 und EP-OS 182 378 werden Copolymerisate aus 3-Hydroxybutansäure und 3-Hydroxypentansäure beschrieben, welche durch mikrobiologische Techniken erzeugt werden. Deren Verwendung als Verpackungsmaterial wird vorgeschlagen. In EP-OS 204 931 und WO 8607250 werden biologisch resorbierbare Objekte aus Homo-poly-3-hydroxybutanoat aufgezeigt.

Die Verwendung von Copolymerisaten von 3-Hydroxyalkylcarbonsäuren als resorbierbares Knochenersatzmaterial wird in der ICI-Firmenschrift "Biopol", 1987 beschrieben.

Die Polymerisate lassen sich nach dem Schema

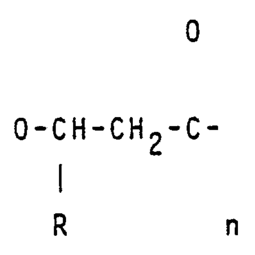

symbolisieren, wobei R die Reste $CH_3$, und $-C_2H_5$, repräsentiert. Die Verteilung der Monomeren in den Copolymerisaten ist statistisch.

n ist eine Zahl von 1500 bis 5000. Es ist möglich und je nach gewünschter Resorptionsgeschwindigkeit gegebenenfalls vorteilhaft, Polymerengemische aus den erfindungsgemäßen Polymerisaten bzw. Copolymerisaten und anderen resorbierbaren Substanzen, z.B. Polyglycolid, Polylactid, Poly-(Dioxanon) einzusetzen. Dabei können die jeweiligen mittleren Molekulargewichte so variiert werden, daß den Erfordernissen des Anwendungsbereiches Rechnung getragen wird.

Die speziellen Materialeigenschaften sind über die Wahl des Polymerisationsgrades und gegebenenfalls des Monomerenverhältnisses steuerbar.

Copolymerisate aus 40 - 98 Gew.-% HB und 2 - 60 Gew.-% HP weisen hohe Flexibilität bei guter Festigkeit auf. Vorteilhaft ist ein Bereich von 70 - 95 Gew.-% HB, und 5 - 30 Gew.-% HP, besonders vorteilhaft 85 - 92 Gew.-% HB und 8 - 15 Gew.-% HP. Das bevorzugte Molekulargewicht ist 10.000 - 500.000 Dalton, bevorzugt 100.000 - 350.000 Dalton.

Die Flexibilität eines Materials mit gegebenem Molekulargewicht kann vorteilhaft durch Zugabe eines Weichmachers beeinflußt werden.

Die Mono- Di- und Triethylester der aliphatischen Hydroxy- bzw. Dihydroxymono-, -di- und tricarbonsäuren, insbesondere der Citronensäure haben sich als Weichmacher gut bewährt, insbesondere in einer Menge von 0,5 - 5,0 Gew.-%, bezogen auf die gesamte Zusammensetzung.

Besonders bevorzugt ist ein Anteil von 2,0 - 3,5 Gew.-% dieser Ester, in Bezug auf die gesamte Zusammensetzung.

Weiterhin ist vorteilhaft, als Weichmacher Polyethylenglykole mit einem mittleren Molekulargewicht von 5.000 - 30.000 einzusetzen, bevorzugt in einem Anteil von 1,0 - 15,0 Gew.-%, bezogen auf die gesamte Zusammensetzung.

Es ist günstig, den polymeren Grundstoffen übliche medizinische Hilfs- und/oder Zusatzstoffe beizufügen, beispielsweise Röntgenkontrastmittel (vorteilhaft $ZrO_2$ oder $BaSO_4$ in einem Gehalt bis zu 50 % des Gesamtgewichts), isotopenmarkierte Zusatzstoffe, physiologisch wirksame Substanzen (z.B. pharmazeutische Wirkstoffe wie Relaxantien, Sedativa, Antibiotika, antivirale Stoffe), Farbstoffe, (z,B. Fluorescein).

Die Einarbeitung dieser Hilfsstoffe erfolgt bevorzugt durch Beimengen zum granulierten Festkörper, zu einer Lösung, einer Suspension oder einer Schmelze des Polymerisats. Dies setzt voraus, daß die gewählten Zusatzstoffe mit den Aufarbeitungsverfahren, welche in einen Arbeitsgang der Formkörperherstellung integriert sein können, verträglich sind.

Bevorzugt in Intestinaltrakt und insbesondere im Verdauungsbereich bieten die erfindungsgemäßen Implantate den Vorteil, daß sie in angemessener Zeit abgebaut werden. Herkömmliches resorbierbares Material vermag den aggressiven Verdauungssekreten zum einen nur kurze Zeit standzuhalten, zum anderen ist seine Resorptionsrate nur sehr unzuverlässig prognostizierbar.

In den nachfolgenden Beispielen seien die erfindungsgemäßen Materialien beispielhaft erläutert, ohne daß dadurch eine Einschränkung auf diese Beispiele erfolgen soll.

Beispiel 1

Copolymerisat:
HB : 95 Gew.-%
HP : 5 Gew.-%
Molekulargewicht 200.000

Beispiel 2

Copolymerisat:
HB : 80 Gew.-%
HP : 20 Gew.-%
Molekulargewicht 230.000

Beispiel 3

Mischung aus 70 Gewichtsteilen Copolymerisat gemäß Beispiel 1 und 30 Gewichtsteilen $ZrO_2$.

Beispiel 4

Mischung aus 70 Gewichtsteilen Copolymerisat gemäß Beispiel 2 und 30 Gewichtsteilen $ZrO_2$.

Die Copolymerisate sind käufliche, mikrobiell nach EP-OS 204 442 hergestellte Produkte.

Die Polymerisate gemäß den Beispielen 1 und 2 können durch Foliengießverfahren in Folienform gebracht werden. Dazu geht man von einer Lösung oder Suspension der Grundmischung in einem Lösungsmittel, vorteilhaft einem leicht verdunstenden Lösungsmittel (z.B. Chloroform) aus, die auf der Platte eines handelsüblichen Foliengießgerätes ausgegossen wird. Das verdunstende Lösungsmittel hinterläßt eine gleichmäßige Folie, die blasenfrei in Dicken von 0,05 bis 2,5 mm erhältlich ist.

Die Mischungen gemäß den Beispielen 3 und 4 werden aufgeschmolzen und auf die Platte des Foliengießgerätes aufgetragen und verstrichen. Nach Erstarren und Abkühlen des Materials kann die fertige Folie abgezogen werden. Schichtdicken von 1 bis 5 mm sind so erhältlich. Besonders bevorzugt ist, derartige Folien durch Extrusionsverfahren anzufertigen.

Um die bei manchen Indikationen zu erwartenden Nekrosen infolge Störungen des osmotischen Austausches zu vermeiden, können die Folien perforiert werden. Die Lochgröße sollte so gewählt werden, daß die Gewebsregionen zu beiden Seiten der Folie nicht durch die Löcher in Kontakt treten können. Als günstig hat sich ein mittlerer Lochdurchmesser von 50 - 2000 my erwiesen, bevorzugte Lochdichte ist 100 - 10.000 Löcher je $cm^2$.

Die Perforation erfolgt nach an sich bekannten Verfahren durch Stanzen oder Druckluftperforation, vorteilhaft durch Laserperforation. Durch Laserperforation werden besonders gleichförmige Löcher mit abgerundeten Kanten erzeugt.

Eine weitere Anwendungsmöglichkeit ist die Verarbeitung der erfindungsgemäßen Polymerisate oder Mischungen zu hohlen Formkörpern, zur Unterstützung hohler Organe. Bevorzugt dabei ist die Anwendung an Speiseröhre, Magen, Duodenum, Darmtrakt und harnleitenden Hohlorganen, aber auch luft- und bluttransportierenden Systemen.

Die erfindungsgemäßen Hohlkörper sollten zumindest eine Öffnung aufweisen. Sie werden bevorzugt in Form eines Ringes, eines Schlauches oder einer Röhre ausgestaltet. Als Implantate dienen sie vorzugsweise der Anastomose.

Eine reliefartige äußere Oberfläche, im einfachsten Falle eine Aufrauhung, vergrößert die Kontaktfläche mit dem geschädigten Organ und bewirkt bessere Haftung. Besonders vorteilhaft sind ringförmige Erhebungen und/oder Vertiefungen, die den Grundkörper umgeben. Derartige Erhebungen sind geeignet, mit dem zu schienenden Organ - vorzugsweise unter Verwendung resorbierbaren Nahtmaterials - vernäht zu werden. Sie dienen als Sperre gegen ein Verrutschen des Hohlkörpers im geschienten Organ. Besonders bevorzugt ist dabei die Ausbildung ringförmiger Wülste, in deren Zwischenräume chirurgische Nähte verlegt werden können. Vorteilhaft sind auch ringförmige Vertiefungen, in die die Nähte gebettet werden können, bzw. über denen das Organ mit Hilfe von Zwirnschlingen oder Klammern eingeschnürt wird.

Ein weiterer Vorteil ringförmiger Erhebungen ist deren Funktion als Abdichtung der Nahtstelle. Konische Erweiterung beziehungsweise Verdickung der Röhrenenden führen in einer weiteren bevorzugten Ausführungsform zu einer verbesserten Abdichtung der Nahtstelle.

Die spezielle Ausgestaltung von Anastomoseröhren als oberflächenstrukturierte Hohlzylinder ist einesteils eine besonders vorteilhafte Ausführung der erfindungsgemäßen chirurgischen Implantate. Andererseits ist sie nicht eingeschränkt auf die Verwendung von Homo- und Copolymerisaten von Hydroxypentansäure und Hydroxybutansäure. Vielmehr kann der Fachmann aus allen toxikologisch vertretbaren resorbierbaren Materialien mit geeigneten physikalischen Eigenschaften Anastomoseröhren der geschilderten Ausgestaltung herstellen. Dabei ist allerdings zu berücksichtigen, daß die Materialien des Standes der Technik nicht über die überlegenen Eigenschaften der Homo- und Copolymerisate aus HB und HP verfügen, insbesondere nicht über deren Resorptionsverhalten im Intestinaltrakt.

In Fig. 1 ist eine vorteilhafte Ausführungsform einer Anastomosehülse dargestellt. Der röhrenförmige Grundkörper A ist in Seitenansicht gezeigt, wobei der Bereich A1 in Aufsicht, der Bereich A2 im Schnitt gezeigt wird. A wird von zwei ringförmigen Wülsten B und B′ an den Röhrenöffnungen umgeben. Der röhrenförmige Grundkörper ist von einem weiteren ringförmigen Wulst C umgeben. Der ganze Körper ist einstückig.

Die Anastomose eines schlauchförmigen Hohlorgans erfolgt vorteilhaft durch Stülpen dessen zertrennter Enden über die Röhre und Vernähen der Kontaktstelle über der durch die Wülste B und C gebildeten Rinne D oder der durch die Wülste C und B′ gebildeten Vertiefung E. Die Wülste B, C und B′ dichten das so geschiente Organ gegen unerwünschten Materialausfluß ab.

Eine weitere vorteilhafte Ausführungsform ist in Fig. 2 abgebildet. Ein röhrenförmiger Grundkörper A ist in Seitenansicht gezeigt, wobei der Bereich A1 in Aufsicht, der Bereich A2 im Schnitt gezeigt wird. A wird von zwei ringförmigen Wülsten C und C′ umgeben. Die Röhrenenden F und F′ sind konisch verbreitert. Vorteilhaft, aber nicht zwingend, ist die Wandstärke des Implantates in der konischen Erweiterung größer auszubilden als auf dem Grundkörper selbst. Das Implantat ist einstückig.

Die Anastomose eines schlauchförmigen Organs erfolgt, indem die zertrennten Enden über die Röhre A gestülpt und über der durch die Wülste C und C′ gebildeten Rinne D vernäht werden. Sowohl die Wülste C und C′ als auch die konischen Röhrenenden F und F′ dienen zur Abdichtung gegen unerwünschten Materialausfluß.

Anastomoseröhren können vorteilhaft durch Spritzguß-oder Extrusionsverfahren aus einer Schmelze des Grundstoffes erhalten werden. Die äußere Gestaltung der Röhren ist durch die Spritzgußform beliebig zu wählen.

Eine weitere Methode zur Fertigung von Anastomoseröhren besteht darin, daß Folien, deren Herstellung vorab beschrieben wurde, zu Röhren gebogen oder gewickelt werden.

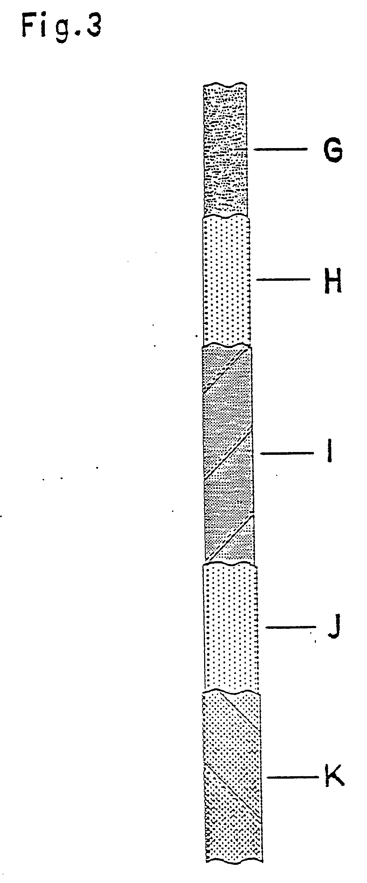

Wie in Fig. 3 beispielhaft dargestellt, kann um einen zylindrischen Körper, beispielsweise einen Edelstahlstab G eine Lage dünner Folie, beispielsweise Aluminiumfolie H gewickelt und fixiert werden. Von der erfindungsgemäßen Folie I wird ein Streifen abgeschnitten und in einer schraubenartigen Anordnung so über die Folie H gewickelt, daß benachbarte Lagen einander überlappen. Um ein Abwickeln der Folie zu verhindern, werden die Folienenden auf dem zylindrischen Körper fixiert, beispielsweise mit einer Aluminiumfolie J. Über die fixierende Folie J wird eine Lage Teflonfolie K gelegt und damit über die ganze Länge des Stabes mäßig fest bandagiert.

Die so vorbereitete zylindrische Form wird 3 Minuten auf ca. 250 °C erhitzt.

Nach dem Abkühlen werden J und K vorsichtig entfernt. Die an den Überlappungsstellen verschmolzene Polymerfolie liegt nun als Röhrchen vor, welches noch auf dem Zylinder zu geeigneter Länge zerschnitten werden kann. Danach werden die einzelnen Hülsen mit der darunterliegenden Aluminiumfolie von dem Metallstab abgezogen. Die Folie wird aus den Hülsen entfernt.

Die von ggf. vorhandenen Rückständen der Lösungsmittel befreiten Implantate sind für den operativen Gebrauch nach den üblichen Methoden zu sterilisieren. Günstig hat sich die Bestrahlung mit ionisierender Strahlung, vorteilhaft mit Wellenlängen kleiner als 250 nm, besonders vorteilhaft mit Gammastrahlung oder Elektronenstrahlung erwiesen. Die verwendete Strahlungsdosis sollte im Bereich von 5 - 500 kGy liegen. Die harte Strahlung erzeugt vermutlich Fehlstellen in der Polymertextur, die den Abbau im Körper beschleunigen.

## Ansprüche

1. Resorbierbares chirurgisches Implantat zur Unterstützung der Organ- und Gewebsheilung, dadurch gekennzeichnet, daß es
a) ein Homo- oder Copolymerisat aus 3-Hydroxybutansäure (HB) und
3-Hydroxypentansäure (HP) gegebenenfalls im Gemisch mit anderen resorbierbaren polymeren Substanzen,
b) gegebenenfalls chirurgische und/oder medizinische Hilfs- und Zusatzstoffe
enthält.

2. Implantat nach Anspruch 1, dadurch gekennzeichnet, daß es ein Copolymerisat aus 40 - 98 Gew.-% HB und 2 - 60 Gew.-% HP, vorteilhaft 70 - 95 Gew.-% HB und 5 - 30 Gew.-% HP, besonders bevorzugt 85 -92 Gew-% HB und 8 - 15 Gew-% HP enthält.

3. Implantat nach Anspruch 1, dadurch gekennzeichnet, daß das Copolymerisat ein mittleres Molekulargewicht von 10.000 - 500.000 Dalton, vorteilhaft 100.000 - 350.000 Dalton besitzt.

4. Implantat nach Anspruch 1, dadurch gekennzeichnet, daß die Zusatzstoffe aus der Gruppe der
- physiologisch wirksamen Stoffe
- Kontrastmittel
- Markierungsmittel

- Weichmacher
- Farbstoffe
- antimikrobiell und/oder antiviral wirkenden Stoffe

gewählt werden.

5. Implantat nach Anspruch 1, dadurch gekennzeichnet, daß die Weichmacher gewählt werden aus den Polyethylenglycolen eines mittleren Molekulargewichtes von 5.000 - 30.000 und in einem Anteil von 1,0 - 15,0 Gew,-%, bezogen auf die Gesamtzusammensetzung, vorliegen.

6. Implantat nach Anspruch 1, dadurch gekennzeichnet, daß es mit elektromagnetischer Strahlung einer Wellenlänge kleiner als 250 nm bestrahlt wird.

7. Implantat nach Anspruch 1, dadurch gekennzeichnet, daß es als Folie ausgestaltet ist.

8. Implantat nach Anspruch 6, durch regelmäßige Perforation mit Löchern von 50 - 2000 my Durchmesser und einer Lochverteilung von 100 - 10.000 Löchern je $cm^2$ gekennzeichnet.

9. Verwendung eines Implantats nach Anspruch 6 als Trennfolie für unterschiedliche Gewebsregionen des menschlichen oder tierischen Körpers.

10. Implantat nach Anspruch 1, dadurch gekennzeichnet, daß es im wesentlichen als ring-, schlauch-, oder röhrenförmiger Körper ausgestaltet ist.

11. Implantat nach Anspruch 9, durch eine reliefartige äußere Oberfläche gekennzeichnet.

12. Ring-, schlauch- oder röhrenförmiges Implantat aus resorbierbarem Material, dadurch gekennzeichnet, daß es eine oder mehrere den Grundkörper ringförmig umgebende Erhebungen oder Vertiefungen besitzt.

13. Implantat nach einem-oder mehreren der Ansprüche 9 - 11, durch im wesentlichen konische Erweiterung und/oder Verdickung der Öffnungen des Grundkörpers gekennzeichnet.

14. Verwendung eines Implantats nach einem oder mehreren der Ansprüche 1 - 12, als innere oder äußere Schienungshülse für Anastomosen.

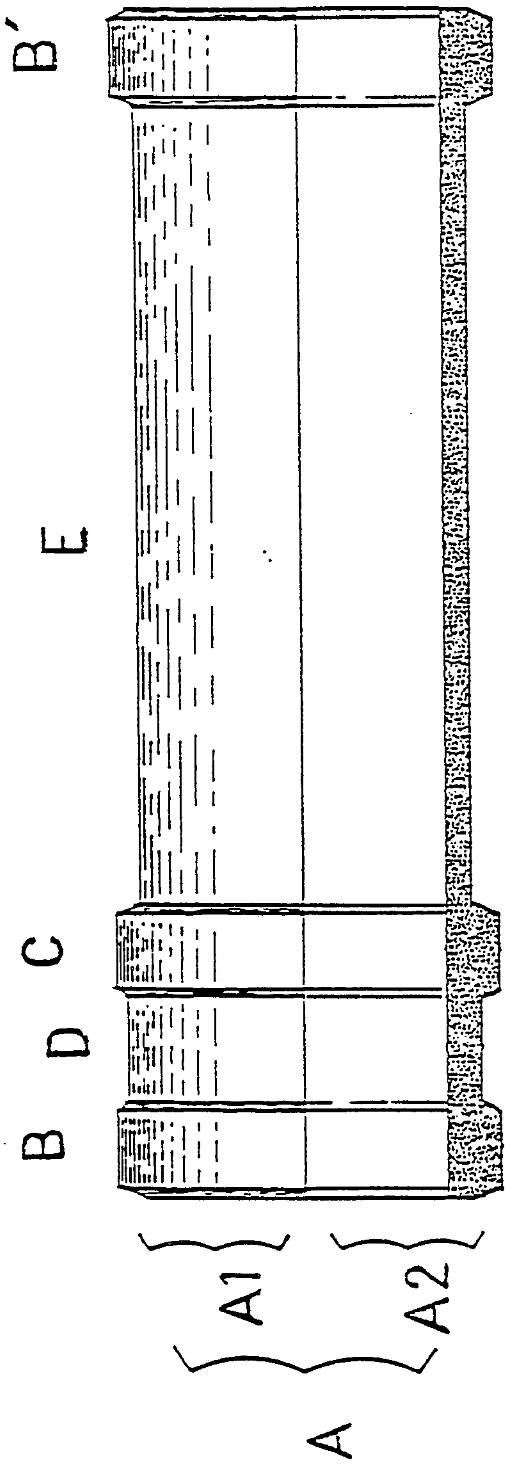


Fig.1

Fig.2

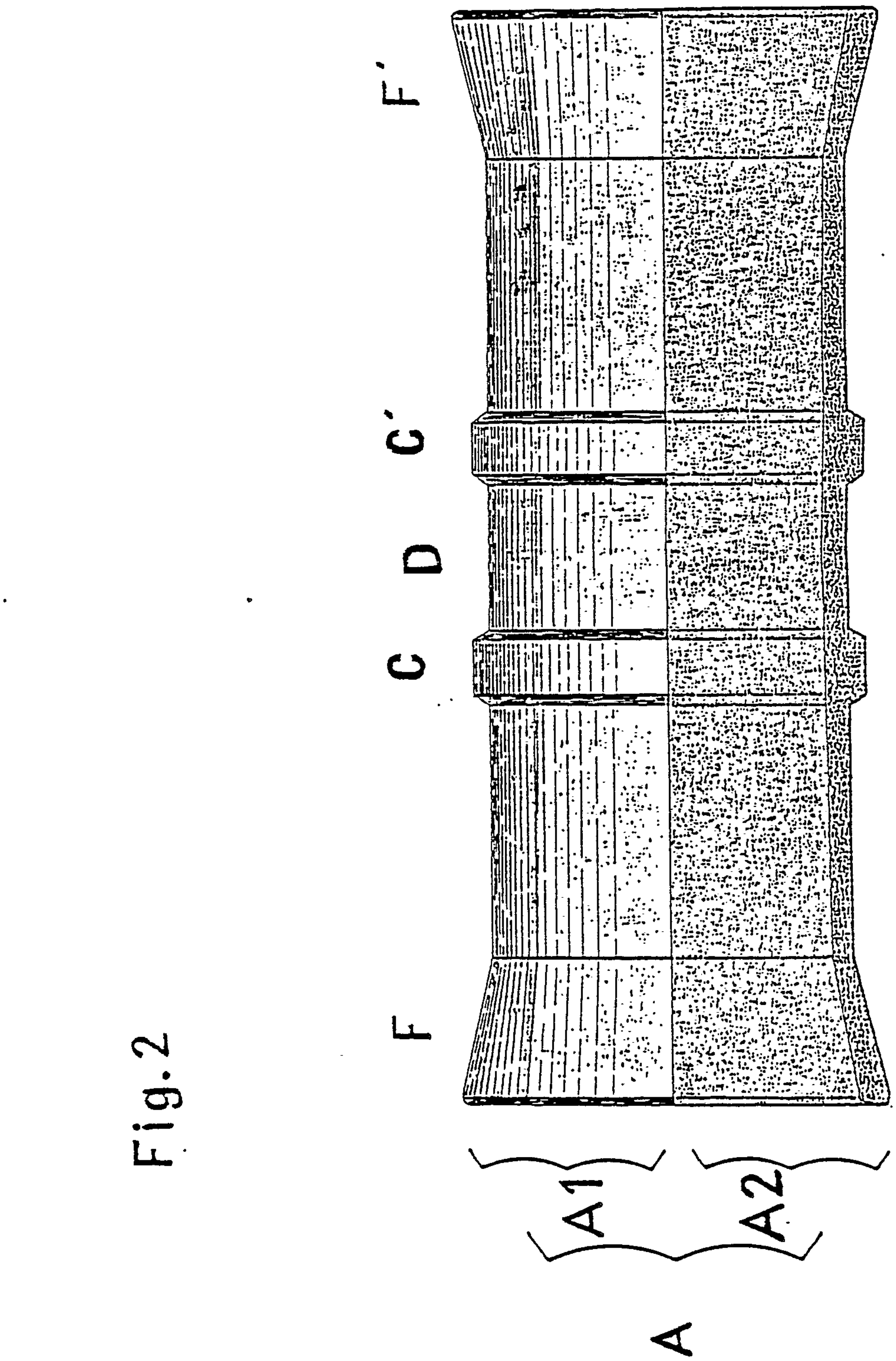

Fig.3